# EUROPÄISCHE PATENTSCHRIFT

(11) **EP 0 364 843 B1**
(45) Veröffentlichungstag und Bekanntmachung des Hinweises auf die Patenterteilung: **27.04.1994**
(21) Anmeldenummer: 89118687.6
(22) Anmeldetag: 07.10.1989
(51) Int. Cl.: C07C 47/347, C07C 49/323, C07C 45/62, C11B 9/00, A61K 7/46

(54) **Isomere Trimethylbicyclo[4.3.0]nonan-Derivate**
Isomers of trimethylbicyclo[4.3.0] nonane derivatives
Isomères de dérivés de triméthylbicyclo[4.3.0]nonane

(30) Priorität: 15.10.1988 DE 3835190
(43) Veröffentlichungstag der Anmeldung: 25.04.1990
(73) Patentinhaber: Henkel Kommanditgesellschaft auf Aktien, 40191 Düsseldorf (DE)
(72) Erfinder: Bruns, Klaus, Dr., D-4150 Krefeld-Traar (DE); Gerke, Thomas, Dr., D-4040 Neuss (DE); Schmitz, Ursula, D-4150 Krefeld-Traar (DE)

(56) Entgegenhaltungen:
- EP-A- 0 021 356
- EP-A- 0 091 033
- EP-A- 0 260 585

## Beschreibung

Die Erfindung betrifft isomere Trimethylbicyclo[4.3.0]nonan-Derivate, ein Verfahren zu deren Herstellung sowie die Verwendung derselben als Riechstoffe.

Aus den deutschen Offenlegungsschriften DE 29 25 622 und DE 32 12 326 ist bekannt, daß isomere Trimethylbicyclo[4.3.0]non-1-en-Derivate der allgemeinen Formel II 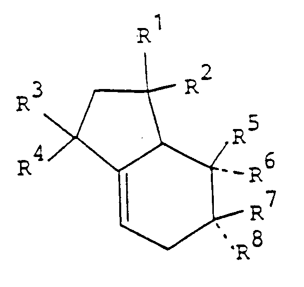
in welcher 3 der Reste R¹ bis R⁴ Methylgruppen und ein Rest ein Wasserstoffatom und 2 der Reste R⁵ bis R⁸ Wasserstoffatome, ein Rest CHO, COCH₃ oder COCH₂CH₃ und ein Rest H, CH₃ oder CH₂CH₃ bedeuten, als Riechstoffe verwendet werden.

Es wurde nun gefunden, daß isomere Trimethylbicyclo[4.3.0]nonan-Derivate, hergestellt durch katalytische Hydrierung von isomeren Trimethylbicyclo[4.3.0]non-1-enen der allgemeinen Form II überraschende und wertvolle Riechstoffeigenschaften besitzen.

Erfindungsgegenstand sind dementsprechend isomere Trimethylbicyclo[4.3.0]nonan-Derivate der allgemeinen Formel I 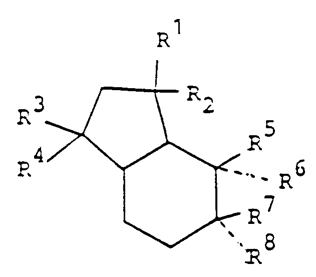
in der 3 der Reste R¹ bis R⁴ Methylgruppen und ein Rest ein Wasserstoffatom und 2 der Reste R⁵ bis R⁸ Wasserstoffatome, ein Rest CHO, COCH₃ oder COCH₂CH₃ und ein Rest Wasserstoff, Methyl oder Ethyl bedeuten.

Ferner ist die Verwendung dieser isomeren Trimethylbicyclo[4.3.0]nonane als Riechstoffe Gegenstand der Erfindung.

Bevorzugt werden solche isomeren Trimethylbicyclo[4.3.0]nonan-Derivate der allgemeinen Formel I, in der 3 der Reste R¹ bis R⁴ Methylgruppen und ein Rest ein Wasserstoffatom und 3 der Reste R⁵ bis R⁸ Wasserstoffatome und ein Rest COCH₃ bedeuten.

Die Herstellung der erfindungsgemäßen isomeren Bicyclo[4.3.0]nonane erfolgt in an sich bekannter Weise durch katalytische Hydrierung isomerer Bicyclo[4.3.0]non-1-ene der allgemeinen Formel II (Houben-Weyl: Methoden der organischen Chemie, 4. Auflage, Band 4/1c, S. 145 - 149, Thieme Verlag 1980). Als Katalysatoren eignen sich beispielsweise Edelmetalle, wie Palladium und/oder Platin, auf Trägermaterialien, wie Bariumsulfat, Kohle oder Aluminiumoxid, nickel- und/oder kupferhaltige Katalysatoren, wie Raney-Nickel und/oder Raney-Kupfer (Houben-Weyl: Methoden der organischen Chemie, 4. Auflage, Band 4/1c, S. 18 - 28, Thieme Verlag Stuttgart 1980). Die Hydrierungen werden in Substanz oder in organischen Lösungsmitteln, beispielsweise Ethanol und/oder Cyclohexan, bei Temperaturen zwischen 20 und 250 °C und bei H₂-Drücken zwischen 10⁵ und 25 · 10⁶ Pa durchgeführt. Nach beendeter Wasserstoffaufnahme wird der Katalysator abfiltriert, gegebenenfalls das Lösungsmittel abdestilliert und der bicyclononanhaltige Rückstand gegebenenfalls unter vermindertem Druck destilliert.

Die als Edukte zur Herstellung der erfindungsgemäßen isomeren Trimethylbicyclo[4.3.0]nonane einzusetzenden isomeren Trimethylbicyclo[4.3.0]non-1-ene der allgemeinen Formel II sind nach den in DE-OS 29 25 622 und DE-OS 32 12 326 beschriebenen Verfahren zugänglich. Als Ausgangsmaterial dient 2,2,4(2,4,4)Trimethylcyclopentanon, das als Isomerengemisch vorliegt. Trimethylcyclopentanon wird in einer Grignardreaktion mit Vinylmagnesiumbromid und anschließender Dehydratisierung mit p-Toluolsulfonsäure zu 1-Vinyl-2,2,4(2,4,4)trimethylcyclopent-1-en umgesetzt. Anschließend wird das erhaltene Diengemisch mit einer äquimolaren Menge oder mit einem geringen Überschuß an dienophilen Aldehyden oder Ketonen nach Diels-Alder bei Temperaturen zwischen 0 und 200 °C und Drücken zwischen 10⁵ und 2 · 10⁷ Pa in die isomeren Trimethylbicyclo[4.3.0]non-1-en-Derivate der allgemeinen Formel II überführt. Als dienophile Aldehyde oder Ketone werden Acrolein, Crotonaldehyd, Ethylacrolein, Methylvinylketon, Ethylvinylketon, Penten-3-on(2) oder Methyl-isopropenylketon, vorzugsweise Methylvinylketon eingesetzt.

Die erfindungsgemäßen Isomerengemische besitzen ein komplexes Geruchsprofil, worin Irisbutter-, Methyljonon-, Tabak- und Holznoten dominieren, und zeichnen sich durch außerordentlich gute geruchliche Nachhaltigkeit aus.

Die erfindungsgemäßen Bicyclononan-Derivate eignen sich zur Herstellung neuer interessanter Riechstoffkompositionen. Bezogen auf die gesamte Komposition liegt der Gehalt der erfindungsgemäßen Riechstoffgemische zwischen 1 und 50 Gew.-%, vorzugsweise zwischen 1 und 25 Gew.-%. Diese Kompositionen können zur Parfümierung von technischen Artikeln, wie Reinigungs- und Desinfektionsmitteln, von Textilbehandlungsmitteln, von Kosmetika aller Art, wie Duftwässern, Cremes, Lotionen, Aerosolen, Toilettenseifen, Schmicken und Lippenstiften sowie in der Extraitparfümerie eingesetzt werden. In den parfümierten Produkten liegt der Gehalt der Riechstoffkompositionen zwischen 2 und 20 Gew.-%.

### Beispiele

### 1. Herstellung von isomerem Acetyl-trimethylbicyclo[4.3.0]nonan

206,3 g (1 Mol) eines Gemisches aus 4Acetyl-7,7,9-trimethyl-bicyclo[4.3.0]non-1-en und 5-Acetyl-7,9,9-trimethyl-bicyclo[4.3.0]non-1-en im Gewichtsverhältnis 1 : 1 wurden in 500 ml Ethanol gelöst und mit 8 g Palladium auf Kohle (Palladiumgehalt 5 Gew.-%) 6 Stunden bei 22 °C mit 3 x 10 ⁶ Pa H₂ gerührt. Nach beendeter Wasserstoffaufnahme wurde der Katalysator abfiltriert, das Lösungsmittel abdestilliert und das Produkt zwischen 58 und 72 °C / 13 Pa destilliert. Die Ausbeute betrug 161 g (78 % der Theorie).
IR (Film) : 1 710/cm (Carbonyl), 1 150/cm (COCH₃), 1 340 - 1 380/cm (gem. Dimethyl, COCH₃)
MS: 208,3 m/e
¹H-NMR (CDCl₃): 0,75 - 1,10 ppm (m, 9H); 2,15 ppm (m, 3H); keine olefinischen Protonen nachweisbar.
Geruch: Irisbutter-, Methyljonon-, Tabak-, Holznote.

### 2. Kompositionsbeispiel

Gew.-Teile bedeutet Gewichtsteile.

| Base für Herren-Eau de toilette | |
|---|---|
| Boisambrene forte (Henkel) | 300 Gew.-Teile |
| isomeres Acetyl-trimethylbicyclo[4.3.0]nonan gemäß Beispiel 1 | 100 Gew.-Teile |
| Linalylacetat | 80 Gew.-Teile |
| α-Hexylzimtaldehyd | 80 Gew.-Teile |
| Lyral (IFF) | 60 Gew.-Teile |
| Terpinylacetat | 50 Gew.-Teile |
| Patchouliöl | 50 Gew.-Teile |
| Galaxolide (IFF) | 50 Gew.-Teile |
| Cumarin | 40 Gew.-Teile |
| Cyclohexylsalicylat (Henkel) | 40 Gew.-Teile |
| Muskatellersalbeiöl | 30 Gew.-Teile |
| Benzylacetat | 30 Gew.-Teile |
| Aldehyd C 12 (Dodecan al,10 Gew.-% in Dipropylenglycol) | 30 Gew.-Teile |
| Zitronenöl | 20 Gew.-Teile |
| Vetiverylacetat | 20 Gew.-Teile |
| Geraniumöl Bourbon | 10 Gew.-Teile |
| Rosenoxid (10 Gew.-% in Diproylenglycol) | 10 Gew.-Teile |
| | 1̅ 0̅0̅0̅ G̅e̅w̅.̅-̅T̅e̅i̅l̅e̅ |

## Patentansprüche

1. Isomere Trimethylbicyclo[4.3.0]nonan-Derivate der allgemeinen Formel I 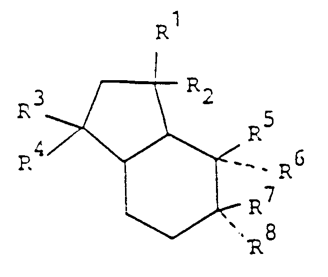 in der 3 der Reste R¹ bis R⁴ Methylgruppen und ein Rest ein Wasserstoffatom und 2 der Reste R⁵ bis R⁸ Wasserstoffatome, ein Rest CHO, COCH₃ oder COCH₂CH₃ und ein Rest Wasserstoff, Methyl oder Ethyl bedeuten.

2. Isomere Trimethylbicyclo[4.3.0]nonan-Derivate nach Anspruch 1 dadurch gekennzeichnet, daß 3 der Reste R⁵ bis R⁸ Wasserstoffatome und ein Rest COCH₃ bedeuten.

3. Verfahren zur Herstellung isomerer Trimethylbicyclo[4.3.0]nonan-Derivate der allgemeinen Formel I 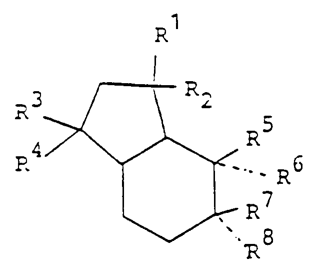 in der 3 der Reste R¹ bis R⁴ Methylgruppen und ein Rest ein Wasserstoffatom und 2 der Reste R⁵ bis R⁸ Wasserstoffatome, ein Rest CHO, COCH₃ oder COCH₂CH₃ und ein Rest Wasserstoff, Methyl oder Ethyl bedeuten, dadurch gekennzeichnet, daß isomere Trimethylbicyclo[4.3.0]non-1-en-Derivate der allgemeinen Formel II 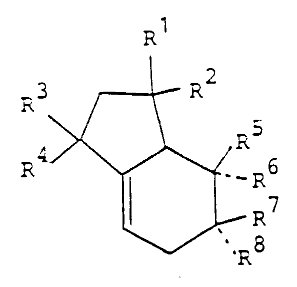 in der 3 der Reste R¹ bis R⁴ Methylgruppen und ein Rest ein Wasserstoffatom und 2 der Reste R⁵ bis R⁸ Wasserstoffatome, ein Rest CHO, COCH₃ oder COCH₂CH₃ und ein Rest Wasserstoff, Methyl oder Ethyl bedeuten, einer katalytischen Hydrierung bei Temperaturen zwischen 20 und 250 °C und H₂-Drücken zwischen 10⁵ und 25 · 10⁶ Pa unterworfen werden.

4. Verfahren nach Anspruch 3, dadurch gekennzeichnet, daß die Hydrierung in Gegenwart edelmetall-, nickel- und/oder kupferhaltiger Katalysatoren durchgeführt wird.

5. Verfahren nach einem oder beiden der Ansprüche 3 bis 4, dadurch gekennzeichnet, daß isomere 4-Acetyl- und/oder 5-Acetyl-7,7,9(7,9,9)-trimethylbicyclo[4.3.0]non-1-ene katalytisch hydriert werden.

6. Verwendung isomerer Trimethylbicyclo[4.3.0]nonan-Derivate der allgemeinen Formel I 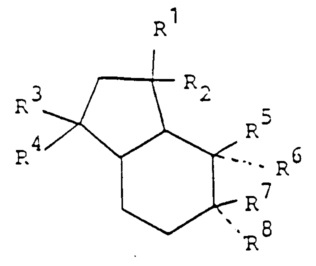 in der 3 der Reste R¹ bis R⁴ Methylgruppen und ein Rest ein Wasserstoffatom und 2 der Reste R⁵ bis R⁸ Wasserstoffatome, ein Rest CHO, COCH₃ oder COCH₂CH₃ und ein Rest Wasserstoff, Methyl oder Ethyl bedeuten, als Riechstoffe.

## Claims

1. Isomeric trimethylbicyclo-[4.3.0]-nonane derivatives corresponding to general formula I: 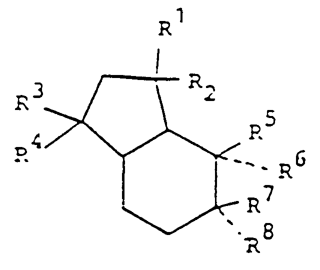 in which three of the substituents R¹ to R⁴ are methyl groups and one is a hydrogen atom and two of the substituents R⁵ to R⁸ are hydrogen atoms, one is CHO, COCH₃ or COCH₂CH₃ and one is hydrogen, methyl or ethyl.

2. Isomeric trimethylbicyclo-[4.3.0]-nonane derivatives as claimed in claim 1, characterized in that three of the substituents R⁵ to R⁸ are hydrogen atoms and one is COCH₃.

3. A process for the preparation of trimethylbicyclo-[4.3.0]-nonane derivatives corresponding to general formula I: 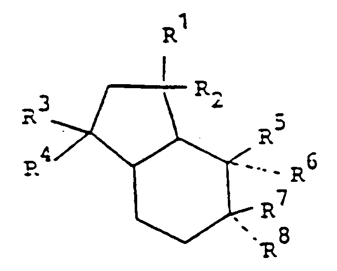 in which three of the substituents R¹ to R⁴ are methyl groups and one is a hydrogen atom and two of the substituents R⁵ to R⁸ are hydrogen atoms, one is CHO, COCH₃ or COCH₂CH₃ and one is hydrogen, methyl or ethyl,
characterized in that isomeric trimethylbicyclo-[4.3.0]-non-1-ene derivatives corresponding to general formula II: 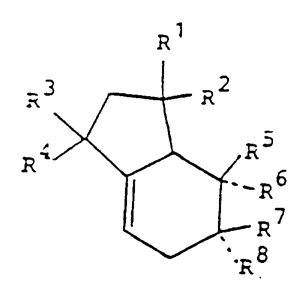 in which three of the substituents R¹ to R⁴ are methyl groups and one is a hydrogen atom and two of the substituents R⁵ to R⁸ are hydrogen atoms, one is CHO, COCH₃ or COCH₂CH₃ and one is hydrogen, methyl or ethyl,
are catalytically hydrogenated at temperatures of 20 to 250°C under hydrogen pressures of 10⁵ to 25 · 10⁶ Pa.

4. A process as claimed in claim 3, characterized in that the hydrogenation is carried out in the presence of catalysts containing noble metals, nickel and/or copper.

5. A process as claimed in one or both of claims 3 to 4, characterized in that isomeric 4-acetyl- and/or 5-acetyl-7,7,9(7,9,9)-trimethylbicyclo-[4.3.0]-non-1-enes are catalytically hydrogenated.

6. The use of isomeric trimethylbicyclo-[4.3.0]-nonane derivatives corresponding to general formula I: 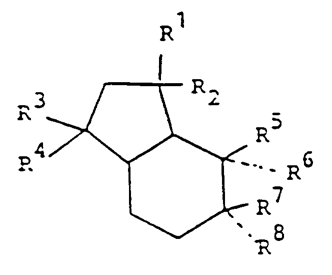 in which three of the substituents R¹ to R⁴ are methyl groups and one is a hydrogen atom and two of the substituents R⁵ to R⁸ are hydrogen atoms, one is CHO, COCH₃ or COCH₂CH₃ and one is hydrogen, methyl or ethyl,
as perfumes.

## Revendications

1. Isomères de dérivés du triméthylbicyclo[4.3.0]nonane de formule générale I : 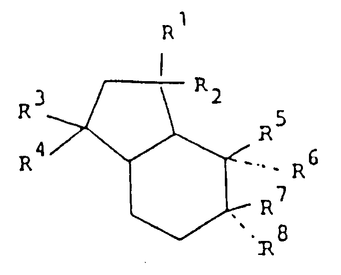 dans laquelle 3 des restes R¹ à R⁴ sont des groupes méthyle et un reste est un atome d'hydrogène et 2 des restes R⁵ à R⁸ sont des atomes d'hydrogène, un reste est CHO, COCH₃ ou COCH₂CH₃ et un reste est un hydrogène, un méthyle ou un éthyle.

2. Isomères de dérivés du triméthylbicyclo[4.3.0]nonane selon la revendication 1, caractérisés en ce que 3 des restes R⁵ à R⁸ représentent des atomes d'hydrogène et un reste COCH₃.

3. Procédé de préparation d'isomère de dérivés de triméthylbicyclo[4.3.0]nonane de formule générale I : 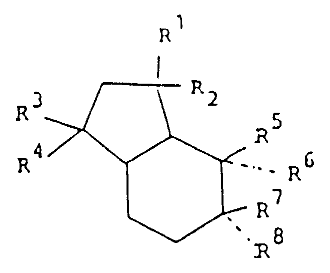 dans laquelle 3 des restes R¹ à R⁴ représentent des groupes méthyle et un reste un atome d'hydrogène et 2 des restes R⁵ à R⁸ des atomes d'hydrogène, un reste représente CHO, COCH₃ ou COCH₂CH₃ et un reste représente un hydrogène, un méthyle ou un éthyle, caractérisé en ce qu'on soumet à une hydrogénation catalytique à des températures comprises entre 20 et 250°C sous des pressions de H₂ comprises entre 10⁵ et 25.10⁶ Pa des isomères de dérivés du triméthylbicyclo[4.3.0]non-1-ène de formule générale II : 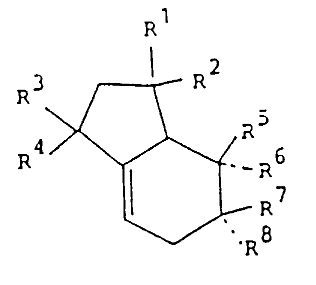 dans laquelle 3 des restes R¹ à R⁴ sont des groupes méthyle et un reste est un atome d'hydrogène et 2 des restes R⁵ à R⁸ sont des atomes d'hydrogène, un reste est CHO, COCH₃ ou COCH₂CH₃ et un reste est un hydrogène, un méthyle ou un éthyle.

4. Procédé selon la revendication 3, caractérisé en ce qu'on réalise l'hydrogénation en présence de catalyseurs contenant un métal précieux, du nickel et/ou du cuivre.

5. Procédé selon l'une ou les deux revendications 3 à 4, caractérisé en ce qu'on hydrogène catalytiquement des isomères de 4-acétyl et/ou 5-acétyl-7,7,9-(7,9,9)-triméthylbicyclo[4.3.0]non-1-ène.

6. Utilisation d'isomères de dérivés du triméthylbicyclo[4.3.0]nonane de formule générale I : 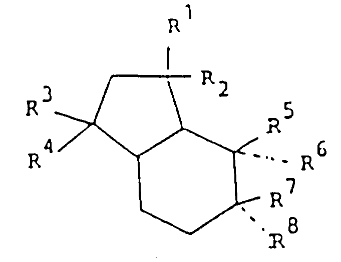 dans laquelle 3 des restes R¹ à R⁴ sont des groupes méthyle et un reste est un atome d'hydrogène et 2 des restes R⁵ à R⁸ sont des atomes d'hydrogène, un reste est CHO, COCH₃ ou COCH₂CH₃ et un reste est un hydrogène, un méthyle ou un éthyle, comme matières odorantes.
